# FASCICULE DE BREVET EUROPEEN

(11) **EP 2 120 834 B1**
(45) Date de publication et mention de la délivrance du brevet: **04.09.2013**
(21) Numéro de dépôt: 08716813.4
(22) Date de dépôt: 12.02.2008
(51) Int. Cl.: A61H 9/00, A61H 23/02, A61H 7/00, A61N 1/04, A61N 1/32, A61N 1/36

(54) **DISPOSITIF POUR LE TRAITEMENT DE LA CELLULITE ET DES MASSES GRAISSEUSES**
VORRICHTUNG ZUR BEHANDLUNG VON CELLULITIS UND FETTMASSE
DEVICE FOR TREATING CELLULITE AND FATTY MASSES

(30) Priorité: 12.02.2007 FR 0753195
(43) Date de publication de la demande: 25.11.2009
(73) Titulaire: Kleinsinger, Alain, 92200 Neuilly-sur-Seine (FR)
(72) Inventeur: Kleinsinger, Alain, 92200 Neuilly-sur-Seine (FR)
(74) Mandataire: Regimbeau
(86) Numéro de dépôt international: PCT/EP2008/051677
(87) Numéro de publication internationale: WO 2008/101839

(56) Documents cités:
- EP-A- 1 386 597
- WO-A-2004/080147
- WO-A-2006/089968
- DE-A1- 19 800 416

## Description

L'invention concerne un dispositif pour le traitement de la cellulite et des masses graisseuses.

La cellulite se définit comme une hydro-lipodystrophie, c'est-à-dire une infiltration d'eau, due à la dilatation des capillaires sanguins, et une accumulation de graisses, accompagnée d'une modification morphologique des cellules graisseuses (adipocytes) qui entraîne un épaississement et une désorganisation du tissu fibreux.

Un oedème se forme, augmente la pression d'eau et comprime les fibres et les cellules ainsi que les vaisseaux sanguins. La circulation veineuse et lymphatique est affectée entraînant un état "d'asphyxie" du tissu conjonctif et son vieillissement prématuré, accompagné de fibrose avec épaississement cutané, responsables de l'aspect caractéristique en «peau d'orange».

La cellulite est localisée dans des régions prédisposées du corps d'un sujet, notamment au niveau des fesses, des hanches et des cuisses.

Conventionnellement, la méthode usuelle pour traiter la cellulite est la liposuccion. On utilise des canules qui pénètrent dans le corps du sujet afin d'aspirer les cellules graisseuses sous-cutanées dans les régions précitées du corps du sujet.

Cependant, cette méthode est invasive, destructive, douloureuse et présente des risques pouvant aller jusqu'à la mort du sujet.

D'autres alternatives à la liposuccion existent pour résoudre ce problème esthétique et traiter l'accentuation des tissus adipeux, par exemple en activant la circulation veineuse locale, en dissolvant la graisse dans les adipocytes ou encore en désorganisant les amas de cellules graisseuses.

On peut citer ainsi l'utilisation de crèmes pour dissoudre les cellules graisseuses ou encore de capsules à ingérer afin d'améliorer le drainage lymphatique des déchets lipidiques, mais ces traitements sont peu efficaces.

On connaît également l'utilisation de dispositifs à ultrasons. La génération d'ultrasons basse fréquence dans le corps du sujet permet de transmettre des vibrations acoustiques aux cellules graisseuses, ce qui active l'exocytose lipidique des adipocytes c'est à dire le rejet des graisses hors des cellules graisseuses.

Cependant, bien que les ultrasons aient une affinité élevée pour le tissu adipeux, il faut limiter leur intensité pour ne pas provoquer un échauffement des tissus, particulièrement sur la peau. La majorité des dispositifs à ultrasons actuels génèrent alors une énergie réduite à transmettre aux tissus, ce qui limite l'efficacité du traitement.

Les graisses expulsées sont ensuite drainées par le système lymphatique et veineux du sujet et éliminées par voie naturelle.

Cependant, ce drainage est souvent insuffisant et une partie des lipides stagnent dans les espaces interstitiels des cellules graisseuses et sont progressivement réabsorbés par ces dernières.

Ces alternatives à la liposuccion n'ont qu'une action temporaire sur la cellulite, ce qui ne donne pas satisfaction.

Un dispositif selon le préambule de la revendication 1 est décrit dans le document WO 2006/089968 A.

Un but de la présente invention est de fournir une alternative efficace, fiable, économique, non invasive et dépourvue de risque, à la méthode de liposuccion dans le traitement de la cellulite et des masses graisseuses.

Un autre but de la présente invention est de proposer un dispositif de traitement de la cellulite et des masses graisseuses qui augmente et accélère l'exocytose lipidique des cellules graisseuses.

Un autre but de la présente invention est de proposer un dispositif de traitement de la cellulite et des masses graisseuses qui augmente l'énergie transmise aux tissus adipeux tout en restant en deçà des limites réglementaires d'intensité d'émissions d'ultrasons.

Un autre but de la présente invention est de proposer un dispositif de traitement de la cellulite et des masses graisseuses qui permet de limiter toute accumulation locale même temporaire des lipides expulsés des cellules graisseuses.

Un autre but de la présente invention est de proposer un dispositif de traitement de la cellulite et des masses graisseuses qui améliore la persistance des résultats du traitement dans le temps.

Un autre but de la présente invention est de proposer un dispositif de traitement de la cellulite et des masses graisseuses qui peut traiter les sujets quelle que soit l'élasticité de leur peau.

Un autre but de la présente invention est de proposer un dispositif de traitement de la cellulite et des masses graisseuses qui propose un traitement indolore.

Ces buts sont atteints selon l'invention grâce à un dispositif pour le traitement de la cellulite et des masses graisseuse selon la revendication 1. Le dispositif comprend des moyens pour former un volume de tissu adipeux sur une zone à traiter du corps d'un sujet, des moyens pour générer des ultrasons et des moyens pour générer des impulsions électriques sur le volume de tissu adipeux ainsi formé.

Les moyens pour générer des ultrasons et les moyens pour générer des impulsions électriques sont supportés par une membrane souple destinée à être disposée en contact avec le volume de tissu adipeux formé.

D'autres caractéristiques, buts et avantages de l'invention apparaîtront à la lecture de la description détaillée qui va suivre, laquelle est purement illustrative et non limitative et doit être lue au regard des dessins annexés sur lesquels :
- La figure 1 représente une vue schématique d'un dispositif de traitement de la cellulite et des masses graisseuses selon l'invention;
- La figure 2 représente un mode de réalisation de moyens de traitement en trois dimensions du dispositif de la figure 1;
- La figure 3 représente un autre mode de réalisation de moyens de traitement en trois dimensions du dispositif de la figure 1;

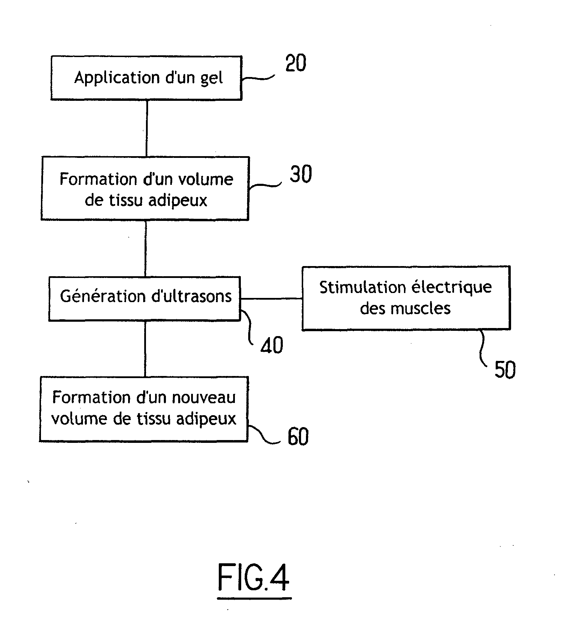

- La figure 4 est un schéma synoptique d'un procédé de traitement de la cellulite et des masses graisseuses mis en oeuvre par le dispositif de la figure 1.

### 1. Structure d'un dispositif pour le traitement de la cellulite et des masses graisseuses

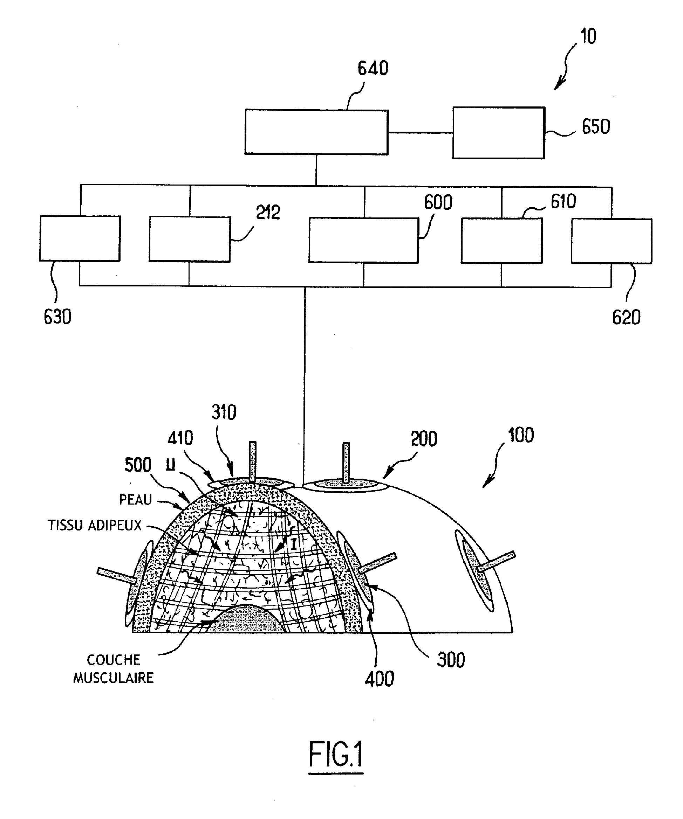

Un dispositif 10 destiné à traiter la cellulite et les masses graisseuses est illustré sur la figure 1.

Il comprend des moyens pour traiter en trois dimensions 100 une zone à traiter du corps d'un sujet, lesdits moyens 100 comprenant des moyens 200 pour former un volume de tissu adipeux à traiter, des moyens 300 pour générer des ultrasons et des moyens 400 pour générer des impulsions électriques placés sur le volume de tissu adipeux ainsi formé.

Les moyens 300 pour générer les ultrasons et les moyens 400 pour générer les impulsions électriques sont placés et répartis sur une membrane souple 500 présentant une certaine élasticité.

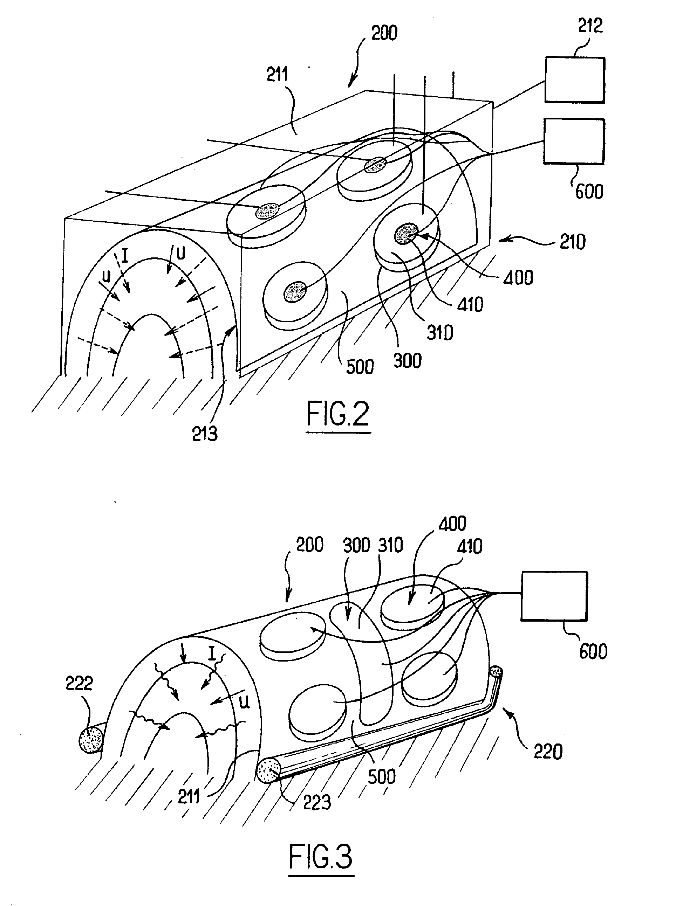

Elle est destinée à venir tapisser l'intérieur des moyens 200 de formation du volume de tissu adipeux permettant ainsi d'appliquer les moyens pour générer les ultrasons et les impulsions électriques 200 et 300 sur la peau du sujet et, par conséquent, sur le volume de tissu adipeux formé comme cela sera décrit plus loin en relation avec les figures 2 et 3.

Plus précisément, sur cette membrane 500 sont placés des électrodes cutanées 410 destinées à conduire dans le volume considéré des impulsions électriques I et des transducteurs cutanés 310 destinés à faire passer dans le volume considéré des ondes ultrasonores U basse fréquence.

L'utilisation des ultrasons U et des impulsions électriques I peut être faite ainsi localement sur le volume de tissu adipeux formé de manière simultanée, successivement ou en alternance pour traiter la cellulite et les masses graisseuses.

Les ultrasons basse fréquence U permettent de transmettre des vibrations aux cellules graisseuses de la couche graisseuse qui activent le phénomène de dissolution de graisse de ces cellules ainsi que l'exocytose lipidique de ces dernières c'est à dire le rejet des graisses hors des cellules.

La possibilité d'associer sur un même volume de tissu adipeux une application d'ultrasons U et d'impulsions électriques I, permet d'améliorer ces résultats.

D'une part, l'envoi de séquences d'impulsions électriques I permet d'améliorer l'efficacité de l'action des ultrasons U basse fréquence sur l'exocytose lipidique des cellules graisseuses.

En effet, l'envoi d'impulsions électriques I permet d'évacuer l'eau qui stagne dans le volume de tissu adipeux traité. Le traitement d'ultrasons U basse fréquence qui aurait eu une action dispersée sur le milieu lipidique et le milieu aqueux présent peut alors se concentrer sur le milieu lipidique et être plus efficace.

D'autre part, l'utilisation de séquences d'impulsions électriques spécifiques I augmente également le débit sanguin du système veineux et active localement la circulation lymphatique qui stimule le drainage lymphatique permettant de réduire l'infiltration excessive d'eau et d'évacuer les rejets lipidiques des cellules vers le foie qui les éliminent.

L'association des séquences d'ultrasons U et de stimulation électrique des muscles provoque alors, au niveau du volume de tissu adipeux traité, une série d'exocytoses de lipides de cellules graisseuses et de drainages qui permet d'obtenir l'évacuation des rejets lipidiques au fur et à mesure du traitement.

On limite ainsi toute accumulation locale même temporaire des lipides qui risqueraient alors d'être réabsorbés par les cellules graisseuses de la couche graisseuse.

Les résultats obtenus sur le traitement de la cellulite et des masses graisseuses du sujet sont ainsi fortement améliorés et durables.

### Ensemble transducteurs /électrodes

Comme indiqué précédemment un ensemble de transducteurs 310 et d'électrodes 410 est disposé sur les moyens 200 formant le volume de tissu adipeux à traiter grâce à la membrane souple 500 afin d'être appliqués sur le volume de tissu adipeux considéré.

De préférence, cet ensemble comprend au moins deux électrodes 410 et deux transducteurs 310 tels qu'illustrés sur les figures 2 et 3.

Les électrodes 410 conduisant les trains d'impulsions électriques I et les transducteurs 310 conduisant les ultrasons U sont alternés sur la membrane souple 500 formant divers motifs.

Ainsi, comme illustré sur la figure 2, les électrodes 410 et les transducteurs 310 peuvent être placés de manière à former des cercles concentriques juxtaposés. Par exemple, les électrodes 410 entourent les transducteurs 310 ou inversement.

Suivant un autre mode de réalisation illustré sur la figure 3, ils sont placés de manière à former un motif en nid d'abeille.

Selon un autre mode de réalisation, la membrane élastique 500 peut former les électrodes 410 dans laquelle des orifices traversants permettent le passage des transducteurs 310.

De plus, de préférence, les transducteurs 310 sont disposés sur la membrane élastique 500 de façon à être à équidistance les uns des autres afin d'optimiser la répartition des ondes ultrasonores U dans les tissus traités.

Par ailleurs, tel qu'illustré sur les figures 1 à 3, l'ensemble des transducteurs 310 et des électrodes 410 est relié à un ou plusieurs générateurs 600 destinés à fournir les impulsions électriques I et à alimenter les transducteurs fournissant les ultrasons U.

Ce générateur 600 fournit des impulsions électriques I par trains avec un courant électrique défini comme un courant alternatif pulsé.

Le courant électrique appliqué à travers les électrodes 410 présente une intensité comprise entre 5µA et 1mA.

Le générateur 600 peut également permettre aux différentes sources d'ultrasons que sont les transducteurs 310 l'envoi d'ondes ultrasonores basses fréquences en phase. Alternativement, on peut prévoir que cette fonction soit assurée par un autre générateur (non-représenté).

Afin d'éviter l'échauffement de la peau du sujet, les ultrasons U sont envoyés par trains de pulsations, comme le courant électrique.

La fréquence des ultrasons basses fréquences est comprise de préférence entre 27Mhz et 300Mhz et, plus précisément entre 100Mhz et 300 Mhz, car à ces fréquences les effets des ondes ultrasonores s'additionnent.

La puissance d'émissions des ultrasons basses fréquences est quant à elle définie pour être inférieure à 3 Watts/cm2 conformément à la législation en vigueur pour éviter les lésions cutanées du sujet.

Il est à noter que les électrodes 410 et/ou les transducteurs 310 peuvent être à usage unique, remplacés à chaque traitement par mesure d'hygiène.

Un gel ou une crème peuvent également être utilisés pour assurer un parfait contact avec l'épiderme du corps du sujet traité.

La membrane 500 peut-être recouverte d'un gel et/ou d'un adhésif et peut-être à usage unique.

### Moyens pour former un volume de tissu adipeux

Le traitement de la cellulite et des masses graisseuses en trois dimensions réalisé grâce aux moyens de formation de volume de tissu adipeux 200 offrent la possibilité d'utiliser plusieurs sources d'ultrasons en phase 310 conduisant des ondes ultrasonores convergeant vers une même zone cible de tissu adipeux.

Dans la mesure où le générateur délivre des signaux en phase ou avec une faible différence de phase à ces sources d'ultrasons 310, les effets des ondes ultrasonores U s'additionnent dans la zone considérée, ce qui génère une plus grande énergie focalisée sur le volume de tissu adipeux traité.

Tout en gardant une intensité inférieure aux limites réglementaires d'intensité d'émission d'ultrasons, l'énergie générée au niveau du volume de tissu adipeux traité est beaucoup plus importante à intensité égale rendant le traitement plus efficace.

La somme des ondes ultrasonores U favorise ainsi l'exocytose des lipides au sein du volume de tissu adipeux.

Afin d'optimiser les effets des ondes ultrasonores U générées en phase, il est possible de disposer de manière adaptée sur les moyens de formation du volume de tissu adipeux à traiter 200, les transducteurs 310.

Ainsi, ils peuvent être orientés de telle manière que les ondes ultrasonores convergent vers le centre du volume de tissu adipeux formé.

Concernant plus précisément les moyens de formation du volume de tissu adipeux 200, ils peuvent comprendre des moyens d'aspiration 210 (succion) et/ou des moyens de pincement mécanique 220 de tissu adipeux tel qu'illustré respectivement sur les figures 2 et 3.

Avantageusement, ces moyens d'aspiration continue ou séquentielle 210 ou de pincement mécanique 220 contribuent par effet mécanique à expulser les lipides des adipocytes, à activer la circulation sanguine et lymphatique et à réduire la fibrose du tissu conjonctif qui entoure les lobes de tissu adipeux.

Ils vont appliquer une dépression ou une pression mécanique externe sur le volume de tissu adipeux qu'ils mobilisent.

Cette pression ou dépression participe à l'action de fragilisation des membranes des cellules graisseuses et favorise leur éclatement ainsi que le rejet des lipides hors des cellules graisseuses.

Elle permet ainsi d'accélérer et d'améliorer l'exocytose lipidique des cellules graisseuses réalisée par le traitement combiné d'ultrasons U basse fréquence et d'impulsions électriques I. Les moyens d'aspiration 210 ou de pincement mécanique 220 formé par des rouleaux mobiles, qui peuvent être motorisés, permettent également d'améliorer le drainage lymphatique des rejets des lipides.

Tel qu'illustré sur la figure 2, les moyens d'aspiration 210 se présentent sous la forme d'un ensemble comprenant un boîtier 211 alimenté par une pompe à vide 212 pour créer une dépression localisée nécessaire à l'aspiration du tissu adipeux.

Au cours de cette dépression, les vaisseaux sanguins se dilatent et transportent via le sang les graisses pour les évacuer.

De préférence, la dépression, réglable par l'utilisateur, est comprise entre 150 et 300 millibars.

Le boîtier 211 présente une chambre 213 interne ouverte sur sa face destinée à être en contact avec la peau du sujet.

Cette chambre 213 s'étend transversalement sur toute la longueur du boîtier 211.

Elle peut être de forme semi-cylindrique ou encore demi-sphérique.

De préférence, elle présente un diamètre interne inférieur à 8 cm.

Toutefois, d'autres variantes de réalisation concernant la forme ou les dimensions de la chambre 213 peuvent être prévues.

La membrane souple 500 comprenant l'ensemble transducteurs 310/électrodes 410 est destinée à venir tapisser la concavité de la chambre 213 pour être en contact avec la peau du sujet.

Afin d'augmenter les effets des ultrasons U générés en phase, les transducteurs 310 de la membrane 500 sont orientés de telle manière que les ondes ultrasonores U convergent vers l'axe central du semi-cylindre ou le centre de la demi-sphère du volume de tissu adipeux formé après aspiration ou pincement.

Par ailleurs, on peut retirer tout transducteur 310 de la membrane 500 dirigé perpendiculairement à la surface de la peau non traitée du sujet afin d'éviter d'envoyer des ondes ultrasonores U vers ses organes internes. Les autres transducteurs 300 sont suffisamment nombreux pour générer une énergie importante au sein du volume traité

Par ailleurs, une variante de réalisation prévoit également des moyens de déplacement automatique des moyens d'aspiration 210 ou de pincement 220.

Ainsi, des rouleaux commandés par des moyens appropriés peuvent être placés sur la périphérie externe de la chambre 213 en contact avec la peau du sujet. Ces rouleaux déplacent les moyens d'aspiration 210 à des intervalles de temps prédéfinis pour changer de volume de tissu adipeux à traiter.

Tel qu'illustré sur la figure 3, les moyens de pincement mécanique 220 comprennent, quant à eux, une plaque 221 souple de forme rectangulaire délimitée par deux rouleaux 222 et 223 sur ces extrémités longitudinales opposées.

Les moyens de pincement mécanique 220 permettent à l'utilisateur de délimiter un volume de tissu adipeux à traiter par un pli de peau par pincement mécanique de la plaque 221 à l'aide des rouleaux 222 et 223.

La membrane 500 souple comprenant l'ensemble transducteurs 310 /électrodes 410 est destinée à venir tapisser la face de la plaque 221 placée en contact avec la peau du sujet.

Dans une variante de réalisation du dispositif 10, on peut prévoir d'utiliser en alternance les moyens d'aspiration 210 et les moyens de pincement mécanique 220 pour réaliser le volume de tissu adipeux à traiter.

Par ailleurs, les moyens de formation de volume de tissu adipeux 200 permettent également d'augmenter l'effet des ultrasons U sur les cellules graisseuses en utilisant, tel que mentionné précédemment, une puissance d'émission des ultrasons inférieure à la limite tolérée sur le corps de 3 Watts /cm2.

Ils permettent également d'utiliser des transducteurs 310 de plus petite taille en conservant une bonne efficacité de traitement, réduisant ainsi le coût de production du dispositif 10.

### Autres moyens du dispositif

Par ailleurs, le dispositif 10 peut comprendre divers capteurs ou dispositifs de contrôle tel qu'illustré sur la figure 1.

Ainsi, il peut comprendre des moyens de refroidissement 610 du volume de tissu adipeux traité en contact avec l'ensemble transducteurs 310/électrodes 410 de sorte d'obtenir une vasoconstriction et de compenser le réchauffement local dû aux ultrasons U auxquels est soumis ce volume.

Par ailleurs, il peut également comprendre des moyens de réchauffement 620 du volume de tissu adipeux traité.

Ainsi, il est possible de réchauffer et refroidir alternativement le volume de tissu adipeux traité afin d'activer la circulation sanguine.

Cette stimulation thermique permet également d'activer le cycle de transformation chimique d'hydrolyse des triglycérides en acides gras utilisés dans le cycle de l'acide pyruvique ou ils sont transformés en pyruvates et consommées par les fibres musculaires.

De plus, la stimulation thermique alternant la chaleur et le refroidissement libère également des neuromédiateurs comme l'adrénaline et la noradrénaline qui sont captés par des récepteurs des adipocytes, notamment les récepteurs bêta, et les stimulent pour déclencher une lipolyse.

Le dispositif 10 peut comprendre également des moyens de détection de température 630 au niveau du volume de tissu adipeux traité adaptés pour couper le ou les générateurs 600 en cas de température excessive relevée au niveau de la peau du sujet.

Par ailleurs, le dispositif peut contenir des capteurs qui donnent des informations sur l'épaisseur et la densité du volume de tissu à traiter permettant si nécessaire d'ajuster les paramètres du dispositif.

Le dispositif 10 peut également comprendre un processeur 640 pour contrôler l'ensemble des moyens mis en oeuvre ainsi que des moyens d'affichage 650.

Ces moyens d'affichage 650 pourront notamment afficher au cours du traitement la durée du traitement choisie et/ou restante ainsi que les caractéristiques des ultrasons et des impulsions électriques telles que la profondeur de pénétration, la fréquence et/ou le courant, la puissance de la dépression. On peut citer comme exemple non limitatif l'utilisation d'un écran à cristaux liquides.

### 2. Procédé de traitement

On va maintenant décrire un procédé de mise en oeuvre du dispositif 10 pour le traitement de la cellulite et des masses graisseuses présenté et notamment pour le traitement cosmétique.

En premier lieu, dans une première étape 20 facultative, on applique un gel sur la peau du sujet traité afin d'améliorer la transmission des ultrasons U dans les tissus du sujet.

Dans une seconde étape 30, on forme un volume de tissu adipeux à traiter.

Pour cela, on utilise soit les moyens d'aspiration 210 soit les moyens de pincement mécanique 220.

Il est à noter que l'aspiration dont la puissance peut être réglée, doit être limitée dans le temps afin d'éviter le décollement du derme de la peau qui entraînerait, après le traitement, l'apparition d'ecchymoses tels que les suçons.

De préférence, elle dure moins de deux minutes sur une même zone de tissu adipeux traité.

Dans un mode de mise en oeuvre du procédé, la membrane souple 500 qui supporte les transducteurs 310 et les électrodes 410 est au préalable déposée à plat sur la peau. La membrane souple 500 est maintenue sur la peau, par des moyens d'attachement, tels que des adhésifs par exemple.

Les moyens d'aspiration 210 ou les moyens de pincement mécaniques 220 sont positionnés au dessus de la membrane souple 500.

Ainsi, lors de l'aspiration, la membrane souple 500 se conforme au volume formé. Autrement dit, la membrane 500 recouvre le volume semi-cylindrique ou semi-sphérique formé. La membrane 500 permet de maintenir les transducteurs 310 et les électrodes 410 en contact avec la peau pendant l'aspiration.

Lorsque le traitement s'effectue sur un sujet qui a une élasticité tissulaire limitée, dépendant notamment de l'épaisseur de la couche de graisse du sujet, l'aspiration des tissus pour former le volume semi-cylindrique ou semi-sphérique risque d'être douloureuse et le derme peut se déformer.

Grace à la membrane souple (500), la dépression réalisée peut être réduite afin de limiter la tension sur les tissus tout en conservant les électrodes 410 et les transducteurs 310 parfaitement appliqués sur le volume de tissu adipeux à traiter.

La membrane permet ainsi un traitement indolore qui peut se réaliser sur tous les sujets quelle que soit l'élasticité de leur peau.

L'ensemble membrane-transducteurs-électrodes peut être supporté par une structure rigide, par exemple plastique ou métallique, qui peut être fixée, par exemple par vissage ou clipsage sur la cloche ou le cylindre d'aspiration. Pour plus de facilité, les différents fils des transducteurs et électrodes peuvent être rassemblés en une prise unique à connecter à sa contrepartie issue du générateur d'électricité. L'ensemble membrane-transducteurs-électrodes-support peut être identifié, par exemple par une puce RFID, un code barre ou un autre moyen, afin que son utilisation soit reconnue par le système. Ainsi, pour des raison d'hygiène, il est possible de n'autoriser le traitement qu'avec un ensemble membrane-transducteurs-électrodes n'ayant jamais été préalablement utilisé, ou bien ayant été utilisé précédemment uniquement pour traiter le patient qui va recevoir un nouveau traitement. Dans ce cas, la reconnaissance de l'ensemble membrane-transducteurs-électrodes permet d'afficher automatiquement le dossier du patient sur la console de contrôle informatique.

S'il n'y a pas d'aspiration, ou si un pincement prend le relais de l'aspiration, la membrane peut être mise en place avant ou après pincement.

Dans un autre mode de mise en oeuvre, il peut être prévu que la membrane 500 tapisse l'intérieur des moyens 200 de formation du volume. Dans ce cas, la membrane 500 est soit perméable de façon à laisser passer l'air aspiré, soit comprend un ou plusieurs orifices aptes à permettre un passage de l'air vers des bouches d'aspiration des moyens 200.

Les électrodes 410 et les transducteurs 310 appliqués sur la peau du sujet vont ensuite conduire respectivement des séquences d'impulsions électriques I et d'ultrasons U prédéterminées sur le volume de tissu adipeux formé.

Ainsi, dans une étape 40, les transducteurs conduisent des ultrasons U générés à une fréquence comprise entre 20 Mhz et 300 Mhz et à une intensité comprise entre 0,5 et 3 watts/cm² sur le volume de tissu adipeux.

Il est à noter que la fréquence et l'intensité de ces ultrasons U peuvent changer au cours du traitement soit manuellement par choix de l'opérateur, soit automatiquement en fonction des informations d'épaisseur, de densité et de température relevées sur la zone traitée.

Pour améliorer et accélérer l'exocytose des lipides des cellules adipeuses, la stimulation électrique est réalisée en même temps que l'envoi des séquences d'ultrasons (étape 50).

Cependant, elle peut aussi être réalisée successivement ou en alternance.

Un courant est ainsi appliqué à travers les électrodes 410 à une intensité comprise entre 5µA et 1mA sur le volume de tissu à traiter.

Les impulsions électriques I générées stimulent alors les muscles lisses, du volume de tissu adipeux traité.

L'alternance de la contraction et du relâchement des muscles lisses augmente le débit sanguin du système veineux et stimule localement la circulation lymphatique permettant d'évacuer ces rejets de lipides des cellules pour être éliminés.

Des séquences temporelles d'impulsions électriques I sont préférées à une application continue d'impulsions électriques I.

Dans une dernière étape 60, grâce à des moyens de déplacement soit automatiques soit manuels, les moyens de traitement en trois dimensions 100 sont ensuite déplacés vers une autre zone du corps de sujet à traiter.

## Revendications

1. Dispositif (10) pour le traitement de la cellulite et des masses graisseuses, comprenant moyens (200) pour former un volume de tissu adipeux sur une zone à traiter du corps d'un sujet, des moyens (300) pour générer des ultrasons et des moyens (400) pour générer des impulsions électriques sur le volume de tissu adipeux formé, **caractérisé en ce que** le dispositif comprend une membrane souple, les moyens pour générer des ultrasons (300) et les moyens pour générer des impulsions électriques (400) étant supportés par la membrane souple (500) liée aux moyens (200) pour former un volume de tissu adipeux, et adaptée pour que, lorsque le dispositif (10) est disposé sur la zone à traiter du corps d'un sujet, les moyens (300) pour générer des ultrasons et les moyens (400) pour générer des impulsions électriques demeurent au contact du volume de tissu adipeux à traiter.

2. Dispositif selon la revendication précédente, **caractérisé en ce que** les moyens pour générer des impulsions électriques (400) comprennent au moins deux électrodes cutanées (410) destinée à conduire dans le volume de tissu adipeux des impulsions électriques et les moyens pour générer des ultrasons (300) comprennent au moins deux transducteurs cutanés (310) destinés à conduire dans le volume de tissu adipeux les ultrasons, les électrodes (410) et les transducteurs (310) étant disposés sur la membrane souple (500).

3. Dispositif selon la revendication 2, **caractérisé en ce que** les électrodes (410) et les transducteurs (310) sont placés sur la membrane souple (500) de sorte de former un motif en nid d'abeille.

4. Dispositif selon la revendication 2, **caractérisé en ce que** les électrodes (410) et les transducteurs (310) sont placés sur la membrane souple (500) de sorte de former un motif dans lequel les électrodes (410) entourent les transducteurs (310) ou inversement.

5. Dispositif selon l'une des revendications précédentes, **caractérisé en ce que** les moyens pour former un volume de tissu adipeux (200) comprennent des moyens d'aspiration de puissance réglable (210) de tissu adipeux.

6. Dispositif selon l'une des revendications 1 à 4, **caractérisé en ce que** les moyens pour former un volume de tissu adipeux (200) comprennent des moyens de pincement mécanique (220) de tissu adipeux.

7. Dispositif selon l'une des revendications précédentes, **caractérisé en ce que** les moyens pour générer des ultrasons (300) et les moyens pour générer des impulsions électriques (400) sont alimentés par un ou plusieurs générateurs (600).

8. Dispositif selon l'une des revendications précédentes, **caractérisé en ce qu'**il comprend en outre des moyens de refroidissement (610) et / ou des moyens de réchauffement (620) du volume de tissu adipeux à traiter formé.

9. Dispositif selon l'une des revendications précédentes, **caractérisé en ce qu'**il comprend en outre des moyens de détection de température (630) au niveau du volume de tissu adipeux à traiter formé.

10. Dispositif selon l'une des revendications précédentes, **caractérisé en ce qu'**il comprend en outre des moyens de détection d'épaisseur et de densité (630) au niveau du volume de tissu adipeux à traiter formé permettant un ajustement des paramètres des ultrasons et du courant.

## Patentansprüche

1. Vorrichtung (10) zur Behandlung von Cellulitis und Fettmasse, umfassend Mittel (200) zur Bildung eines Fettgewebevolumens auf einer zu behandelnden Körperzone eines Patienten, Mittel (300) zur Erzeugung von Ultraschall und Mittel (400) zur Erzeugung von elektrischen Impulsen auf dem gebildeten Fettgewebevolumen, **dadurch gekennzeichnet, dass** die Vorrichtung eine elastische Membran umfasst, wobei die Mittel zur Erzeugung von Ultraschall (300) und die Mittel zur Erzeugung von elektrischen Impulsen (400) durch die elastische Membran (500) getragen werden, die mit den Mitteln (200) zur Bildung eines Fettgewebevolumens verbunden ist und derart ausgebildet ist, dass, wenn die Vorrichtung (10) auf der zu behandelnden Körperzone eines Patienten angeordnet ist, die Mittel (300) zur Erzeugung von Ultraschall und die Mittel (400) zur Erzeugung von elektrischen Impulsen in Kontakt mit dem zu behandelnden Fettgewebevolumen bleiben.

2. Vorrichtung gemäß dem vorhergehenden Anspruch, **dadurch gekennzeichnet, dass** die Mittel zur Erzeugung von elektrischen Impulsen (400) wenigstens zwei Hautelektroden (410) umfassen, die dazu bestimmt sind, in das Fettgewebevolumen elektrische Impulse zu leiten, und dass die Mittel zur Erzeugung von Ultraschall (300) wenigstens zwei Hauttransduktoren (310) umfassen, die dazu bestimmt sind, in das Fettgewebevolumen Ultraschall zu leiten, wobei die Elektroden (410) und die Transduktoren (310) auf der elastischen Membran (500) angeordnet sind.

3. Vorrichtung gemäß Anspruch 2, **dadurch gekennzeichnet, dass** die Elektroden (410) und die Transduktoren (310) auf der elastischen Membran (500) derart platziert sind, dass sie ein Wabenmuster bilden.

4. Vorrichtung gemäß Anspruch 2, **dadurch gekennzeichnet, dass** die Elektroden (410) und die Transduktoren (310) auf der elastischen Membran (500) derart platziert sind, dass sie ein Muster bilden, in dem die Elektroden (410) die Transduktoren (310) umgeben oder umgekehrt.

5. Vorrichtung gemäß einem der vorhergehenden Ansprüche, **dadurch gekennzeichnet, dass** die Mittel zur Bildung eines Fettgewebevolumens (200) Mittel zum Ansaugen mit regulierbarer Stärke (210) von Fettgewebe umfassen.

6. Vorrichtung gemäß einem der Ansprüche 1 bis 4, **dadurch gekennzeichnet, dass** die Mittel zur Bildung eines Fettgewebevolumens (200) Mittel zum mechanischen Abkneifen (220) von Fettgewebe umfassen.

7. Vorrichtung gemäß einem der vorhergehenden Ansprüche, **dadurch gekennzeichnet, dass** die Mittel zur Erzeugung von Ultraschall (300) und die Mittel zur Erzeugung von elektrischen Impulsen (400) durch einen oder mehrere Generatoren (600) gespeist werden.

8. Vorrichtung gemäß einem der vorhergehenden Ansprüche, **dadurch gekennzeichnet, dass** sie außerdem Mittel zur Abkühlung (610) und/oder Mittel zur Erwärmung (620) des gebildeten zu behandelnden Fettgewebevolumens umfasst.

9. Vorrichtung gemäß einem der vorhergehenden Ansprüche, **dadurch gekennzeichnet, dass** sie außerdem Mittel zur Temperaturmessung (630) bei dem gebildeten zu behandelnden Fettgewebevolumen umfasst.

10. Vorrichtung gemäß einem der vorhergehenden Ansprüche, **dadurch gekennzeichnet, dass** sie außerdem Mittel zur Messung der Dicke und der Dichte (630) bei dem gebildeten zu behandelnden Fettgewebevolumen umfasst, wodurch eine Anpassung der Ultraschall- und Stromparameter ermöglicht wird.

## Claims

1. A device (10) for treating cellulite and fatty masses comprising means (200) for forming a volume of adipose tissue on a zone to be treated of the body of a subject, means (300) for generating ultrasound and means (400) for generating electric pulses on the volume of adipose tissue formed, **characterized in that** the device comprises a supple membrane, the means for generating ultrasound (300) and the means (400) for generating electric pulses are supported by the supple membrane (500) connected to the means (200) for forming a volume of adipose tissue, and adapted so that when the device (10) is placed on the zone to be treated of the body of a subject, the means (300) for generating ultrasound and the means (400) for generating electric pulses remain in contact with the volume of adipose tissue to be treated.

2. The device according to the preceding claim, **characterized in that** the means for generating electric pulses (400) comprise at least two cutaneous transducers (410) designed to guide electric pulses in the volume of adipose tissue and the means for generating ultrasound (300) comprise at least two cutaneous transducers (310) designed to guide ultrasound in the volume of adipose tissue, the electrodes (410) and the transducers (310) being placed on the supple membrane (500).

3. The device according to claim 2, **characterized in that** the electrodes (410) and the transducers (310) are placed on the supple membrane (500) so as to form a honeycomb pattern.

4. The device according to claim 2, **characterized in that** the electrodes (410) and the transducers (310) are placed on the supple membrane (500) so as to form a pattern in which the electrodes (410) surround the transducers (310) or inversely.

5. The device according to one of the preceding claims, **characterized in that** the means for forming a volume of adipose tissue (200) comprise suction means of adjustable power (210) for adipose tissue.

6. The device according to one of claims 1 to 4, **characterized in that** that the means for forming a volume of adipose tissue (200) comprise mechanical pincer means (220) for adipose tissue.

7. The device according to one of the preceding claims, **characterized in that** the means for generating ultrasound (300) and the means for generating electric pulses (400) are powered by one or several generators (600).

8. The device according to one of the preceding claims, **characterized in that** it further comprises cooling means (610) and/or heating means (620) for the volume of formed adipose tissue to be treated.

9. The device according to one of the preceding claims, **characterized in that** it further comprises temperature-detection means (630) at the level of the volume of formed adipose tissue to be treated.

10. The device according to one of the preceding claims, **characterized in that** it further comprises means for detecting thickness and density (630) at the level of the volume of formed adipose tissue to be treated allowing adjustment of the parameters of ultrasound and current.
